# EUROPEAN PATENT SPECIFICATION

(11) **EP 3 737 251 B1**
(45) Date of publication and mention of the grant of the patent: **13.12.2023**
(21) Application number: 18898838.0
(22) Date of filing: 08.01.2018
(51) Int. Cl.: G09B 19/00, A41D 19/00, A61F 5/01, A61Q 9/04, A41D 19/015, A45D 26/00

(54) **SUGARING GLOVE APPARATUS AND TRAINING METHOD**
VORRICHTUNG UND VERFAHREN FÜR SUGARING-HANDSCHUH
APPAREIL EN FORME DE GANT POUR ÉPILATION AU SUCRE ET PROCÉDÉ D'APPRENTISSAGE

(43) Date of publication of application: 18.11.2020
(73) Proprietor: Kennedy, Lina, Williamsville, NY 14221 (US)
(72) Inventor: Kennedy, Lina, Williamsville, NY 14221 (US)
(74) Representative: Gee, Steven William
(86) International application number: PCT/US2018/012728
(87) International publication number: WO 2019/135764

(56) References cited:
- WO-A2-2007/011828
- US-A- 3 918 096
- US-A- 4 706 658
- US-A- 5 628 069
- US-A- 5 628 069
- US-A1- 2009 281 470
- US-A1- 2013 298 338
- US-A1- 2014 128 225

## Description

### FIELD OF INVENTION

The present invention relates to a training device and training method for applying a sugar composition for hair removal.

### BACKGROUND

Traditionally, hair removal has been performed by waxing. Waxing is typically done in a salon by a body wax professional using hot or cold wax applied to the hair which the clients wish to have removed. The waxing products are made of petroleum based resins and chemicals which can be prone to contamination or cross contamination. With hot waxing, there are risks of burning skin, damaging the dermal cells, scarring the follicle mouth or surrounding area and causing pigmentation. The wax will adhere to the skin cells, or any surface it is applied to, making pulling it away painful. When waxing, the unwanted hair is removed against the natural direction of growth which causes undue levels of pain and becomes the major contributor to ingrown hair. Hair trapped under the skin which can also be painful and unsightly and cause infections.

A hair follicle is a skin organ that produces hair. Hair production occurs in phases, including a growth phase (anagen), and cessation phase (catagen), and a resting phase (telogen). Stem cells are principally responsible for the production of hair.

Anagen is the active growth phase of hair follicles. The roots of the hair are dividing rapidly, adding to the hair shaft. During this phase, the hair grows about 1 cm every 28 days. Scalp hair stays in this active phase of growth for 2-7 years. The amount of time the hair follicle stays in the anagen phase is genetically determined. There are other factors that can affect hair growth and hair loss patterns such as disease, medication, stress, and severe dieting. Generally, genes play the major role. At the end of the anagen phase, the follicle goes into the catagen phase.

US Patent 5,628,069 discloses a glove for use in motorcycling, skiing, surfing or the like in which one or more of the finger sleeves and the thumb sleeve have a bistable spring element which is stable in a first configuration corresponding to the finger or thumb in a closed or bent position and is stable in a second configuration corresponding to the finger or thumb in an open or linear position.

### SUMMARY OF INVENTION

The present invention relates to a finger alignment training device as defined in the sole independent claim 1.

"Advanced body sugaring" is a unique and innovative process for the removal of unwanted facial and body hair for men, women, and children alike.

In some examples, the methods of the current invention are performed by trained professionals in advanced body sugaring and skin conditioning treatments through salons and spa therapy facilities. In other examples, the methods may be performed at home.

Sugaring requires an entirely different mind-set and approach than waxing. In the methods described, the all natural sugaring compositions do not adhere to live skin cells, therefore, when the compositions are removed, the composition gently exfoliates dead skin cells and leaves behind freshened skin. There is no after-pain, stickiness, bruising or burning when a proper technique is used.

In one example, the sugaring compositions comprise pure sugar, water, and lemon. They are edible and delicious, and since they are water soluble, can be easily rinsed away if accidentally applied to an area. The sugaring will extract all hair colors and textures. The sugaring methods remove the hair in the natural direction of the hair growth.

When the sugar paste is applied, it seeps into the hair follicle and helps lubricate that hair root. The sugaring process will successfully treat all skin types and colors while improving skin tone and texture. Sugaring will eliminate ingrown hairs and help prevent new ingrown hairs from developing. Since sugaring is done at body temperature, the process eliminates the risk of burning the skin. The treatment adheres to the hair, not to the skin, and lubricates the root for easier removal from the hair root.

These factors, combined with the hair removal in the natural direction of growth, create a much more comfortable experience for everyone.

Sugaring techniques vary in user sensation, skin quality, and hair reduction. Sugaring is an all-natural, edible sugar paste, and is not heated enough to burn the skin. If the treatment process is not performed with ease and comfort, however, then it can become an aggressive treatment to the skin and cause undue pain, bruising, and even skin lifting or tearing.

Applicant has practiced and studied sugaring hair removal since 1991, and developed the Kennedy Theory^{®} to sugaring hair removal which is taught in beauty schools around the world. By analyzing videos of the micro-movements in her sugaring technique, applicant created the Kennedy Sugaring Technique Theory^{®} Using 6 Steps to Perfect Sugaring and began training instructors to teach the technique.

When properly performed, the 6 Step technique provides an efficient and comfortable sugaring procedure. Clients consistently complement the treatment and how their skin looks and feels afterwards.

The 6 steps is a precise methodology, and one aspect of teaching the 6 steps is helping the technicians understand and practice specific orientations of the hand and fingers during the various steps of the process. The specific orientations of the hand and fingers are important both to improve the client experience and to reduce practitioner fatigue. By reviewing each step of a practitioners' technique, an instructor can identify where corrections are required.

A common challenge in teaching the technique is that many trainees are unable to position and to hold their hands and fingers correctly enough to ensure the most gentle and efficient treatments. To address this challenge, applicant invented devices to act as an alignment guide for their fingers, and incorporated the finger alignment devices into training methods. The guides establish ergonomically correct hand and finger positioning that align the practitioner's fingers so that an instructor can better focus on the actual technique. The guides reduce stress on both the teacher and the pupil, and facilitate a more effective learning process.

In one example, the finger alignment training device is a training glove where a bent little finger alignment guide is inserted into a dorsal (back of finger) pocket on the little finger sleeve; a bent ring finger alignment guide is inserted into a dorsal pocket on the ring finger sleeve; and a middle finger alignment guide is inserted into a dorsal pocket on the middle finger sleeve. After the trainee puts the glove on, the middle finger, ring finger, and little finger are bent to a desired orientation and the corresponding alignment guides are bent and hold the trainee's middle finger, ring finger, and little finger in a desired curvature.

In another example, the finger alignment training device is a training glove where a bent little finger alignment guide is inserted into a palmar (front of finger) pocket on the little finger sleeve; a bent ring finger alignment guide is inserted into a palmar pocket on the ring finger sleeve; and a middle finger alignment guide is inserted into a palmar pocket on the middle finger sleeve.

In another example, the finger alignment training device is a training glove where a first bent little finger alignment guide is inserted into a dorsal pocket on the little finger sleeve; a second bent little finger alignment guide is inserted into a palmar pocket on the little finger sleeve; a first bent ring finger alignment guide is inserted into a dorsal pocket on the ring finger sleeve; a second bent ring finger alignment guide is inserted into a palmar pocket on the ring finger sleeve; and a first middle finger alignment guide is inserted into a dorsal pocket on the middle finger sleeve. If desired, a second middle finger alignment guide is inserted into a palmar pocket on the middle finger sleeve.

In another example, the finger alignment training device is a glove with detachable finger alignment guides, where the guides may be removable and attached with hook and loop fastener, double sided tape, snap fit, or other means.

In another example, the finger alignment training device is a glove where finger alignment guides are affixed to the glove.

In other examples, the finger alignment training device is provided as one or more form where finger alignment guides are part of a form.

### Theory

The Kennedy Theory^{®} for sugaring hair removal using Alexandria Professional^{®} (AP) sugar paste formulations in conjunction with the Kennedy Sugaring Technique Theory^{®} is as follows: In one embodiment, the AP sugar paste formulation is applied using the Kennedy Sugaring Technique Application as described in Step 200 below - the "molding process". This application technique allows the sugar paste to penetrate down into the follicle and to lubricate the hair inside as well as to grip the hair and inner root sheath. Basically, the sugar is sticking to everything on the inside of the follicle that it touches.

Taking a hair in the early anagen stage promotes a faster refinement (thinning) and depletion of the hair, so that the amount of hair and difficulty of removal decreases in subsequent sugaring treatments. When a hair is extracted intact and in its natural direction while in the anagen stage, it has a large bulb traveling through the follicle which takes some of the inner root sheath with it as it is being extracted. The inner root sheath also carries matrix cells responsible for helping to grow the hair. We are all born with a predetermined amount of matrix cells, and by depleting the follicle's inner root sheath time and time again, you help to deplete the hairs growth source.

In addition to the Kennedy Theory^{®}, when you extract a hair in the early anagen stage, you are taking the hair while it is still attached to the derma papilla - its feeding source/nourishment from blood vessels.

Applicant theorizes that extracting a hair in the early anagen stage causes the vessels to be cauterized. Cauterization may also occur instantly from time to time whereby there are no more vessels to continue the nourishment to a given follicle. This explains a reason why sometimes a hair never grows back after a single treatment.

Applicant theorizes that sometimes only some of the vessels become cauterized, and that this partial cauterization is the reason why we see instant refinement results and ongoing refinement results. This phenomenon is generally observed if the hair is treated in the early anagen stage which means treatments when the hair is quite short such as ½ mm up to 2 mm. The length of hair also depends primarily on the location of the hair on the body.

These theories extend the Kennedy Theory^{®}, to explain why the Alexandria Professional^{®} System is successful at providing immediate excellent instant staggered growth and hair refinement- all leading to permanent results.

### BRIEF DESCRIPTION OF DRAWINGS

FIG. 1 is a top view of an open right hand.
FIG. 2 is a flow chart illustrating an example sugaring technique for hair removal.
FIG. 3A is a front view illustration of a hand and wrist in the "handshake position" of Step 100 of the flow chart of 2.
FIG. 3B is a side view showing the finger and wrist alignment, and finger separation of FIG. 3A.
FIG. 4 is a front view showing the finger and wrist alignment of the sugar paste "molding" of Step 200 of the flow chart of FIG. 2.
FIG. 5 is a front view showing the finger and wrist alignment of the "relax" wrist dropping of Step 300 of the flow chart of FIG. 2.
FIG. 6 is a front view showing the finger and wrist alignment of the "airplane" release of Step 400 of the flow chart of FIG. 2.
FIG. 7A is a front view showing the finger and wrist alignment of the "roll" of Step 500 of the flow chart of FIG. 2.
FIG. 7B is a front view showing the finger and wrist alignment of the "prepare to flick" of Step 500 of the flow chart of FIG. 2.
FIG. 8 is a front view showing the finger and wrist alignment of the "kick off' of Step 600 of the flow chart of FIG. 2.
FIG. 9 is a front view illustration of a hand and wrist the "handshake position" of Step 100 of the flow chart of 2 with sugar paste.
FIG. 10 is a front view showing the finger and wrist alignment and sugar paste of the sugar paste "molding" of Step 200 of the flow chart of FIG. 2.
FIG. 11 is a front view showing the finger and wrist alignment and sugar paste of the "relax" wrist dropping of Step 300 of the flow chart of FIG. 2.
FIG. 12 is a front view showing the finger and wrist alignment and sugar paste of the "airplane" release of Step 400 of the flow chart of FIG. 2.
FIG. 13 is a front view showing the raised hand of the "kick off" of Step 600 of the flow chart of FIG. 2.
FIG. 14 is a front view showing the return to a handshake position following the "kick off' of FIG. 13.
FIG. 15 is a front view showing the molding following the "kick off" and return to a handshake position of FIG. 14.
FIG. 16 is a front view showing the "roll" and return to a handshake position to "prepare to flick" following the "molding" steps of FIG. 14.
FIG. 17 is a front view showing a "kick off' following the "prepare to flick" of FIG. 16.
FIG. 18 is an illustration of a "snap back" following the "kick off" of FIG. 17.
FIG. 19 is a right-side perspective view a right hand held in a first orientation for applying a sugaring composition.
FIG. 20 is a rear left side perspective view a right hand held in a second orientation for applying a sugaring composition.
FIG. 21 is a top perspective view a right hand held in a third orientation for applying a sugaring composition.
FIG. 22A is a top view of a training glove embodiment.
FIG. 22B is a bottom palm-side view of the training glove embodiment of FIG. 22A.
FIG. 23 summarizes the 6 Steps to Perfect Sugaring.
FIG. 24 illustrates the 6 Steps to Perfect Sugaring.
FIG. 25 is a back view of a second example training glove.
FIG. 26 is a back view of the second example training glove of FIG. 25on a stand.
FIG. 27 is a side view of the second example training glove of FIG. 25 on a stand and showing bent finger positions.
FIG. 28 is a back view of the second example glove of FIG. 25.
FIG. 29 is a front view of the second example glove where sleeves are provided on the front portions of the ring, middle, and little fingers.

### DESCRIPTION OF EMBODIMENT

In this specification, the term "palm" refers to the central region of the anterior part of a hand; and the term "opisthenar" refers to the corresponding area on the posterior part of the hand. The term "thumb" refers to the first digit; the term "index finger" refers to the second digit closest to the thumb; the term "middle finger" refers to the third digit; the term "ring finger" refers to the fourth digit; and the term "little finger" refers to the fifth digit. The term "first knuckle" refers to the major knuckle at the base of a finger; the term "third knuckle" refers to the minor knuckle closest to the fingertip; and the term "second knuckle" refers to the minor knuckle between the first knuckle and the third knuckle.

In this specification, the term "proximal" refers to the direction toward the wrist; the term "distal" refers to the direction away from the wrist toward the fingertips; the term "palmar" refers to the palm and grasping side of the fingers; and the term "dorsal" refers to the portion of the hand and fingers opposite the palmar side.

In this specification, the term "training glove" refers to any combination of materials or mesh that covers at least a portion of the palm or opisthenar of a hand, and which facilitates setting and maintaining a desired orientation of some of the fingers on the hand. The term "finger alignment guide" refers to a shaped or shapable element that provides a reference for a desired orientation of the first, second, and third knuckles of a finger. The term "bent finger alignment guide" refers to a shaped or shapable element that provides a reference for a desired bent orientation of the first, second, and third knuckles of a little finger or ring finger. The term "finger alignment training device" refers to a glove, form, or any combination of elements that provides a reference for a desired bent orientation of at least the first, second, and third knuckles of a little finger and ring finger.

In this specification, the terms "fingertip alignment" or "along a line" refers to a substantially linear alignment of the little finger tip, the ring fingertip, and the middle fingertip. The term "sleeve" refers to a closed or open fingertip portion of a glove that fits over a finger. The term "finger inserts" refers to bendable finger alignment guides which may be inserted into pockets provided on finger sleeves, or provided inside the sleeves.

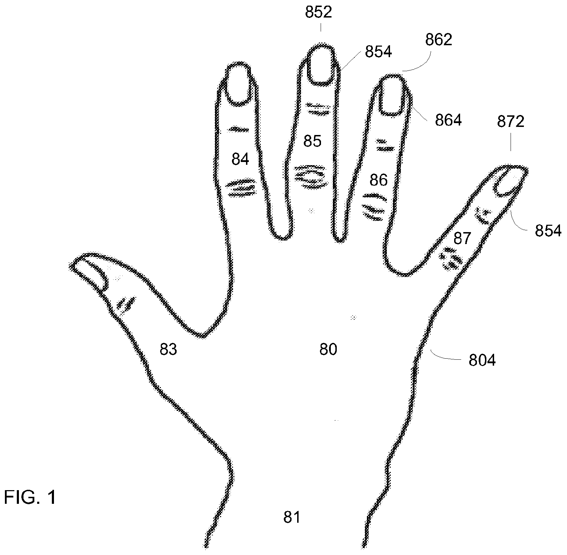

FIG. 1 is a top view of an open right hand **80** with the wrist **82**, the index finger **84**, the middle finger **85**, the ring finger **86**, the little finger **87**, and the thumb **83.**

### Alexandria Professional^{®} sugaring technique

Sugaring treatments cause hair to quickly become less resistant to extraction. With the Alexandria Professional^{®} sugaring technique, the sugar paste is seeped into the follicles and then removed with a "flicking" action in the natural direction of the hair growth. This action actually extracts hair in the early anagen stage while simultaneously removing debris from the follicles and gently exfoliating the skin as well. Extraction of hair in the early anagen stage, when the hair is generally 1/16" (less than 1mm) in length, promotes quicker refinement that leads to diminishment.

Sugaring is an excellent method to refine and diminish hair growth, and simultaneous benefits occur with each treatment. As the sugar paste is molded onto the skin, it naturally seeps into the follicles and sticks to and grips everything it touches inside the follicle including the root of the hair, the debris accumulation from the skin's surface that can become embedded in the follicle, and the inner root sheath carrying matrix cells.

When the quick "flick" action is performed, the sugar paste inside the follicle connected to the sugar paste molded onto the skin is all removed at the same time - taking with it all that is connected to it inside and out. This action is very gentle because the sugar paste acts as a lubricant inside the follicles which allows for a more gentle extraction and allows for the extraction of hair in the natural direction of its growth.

Comodones (blackheads or compacted pores) can also be gently and safely treated with the unique Alexandria Professional sugaring technique.

### 6 Step sugaring technique

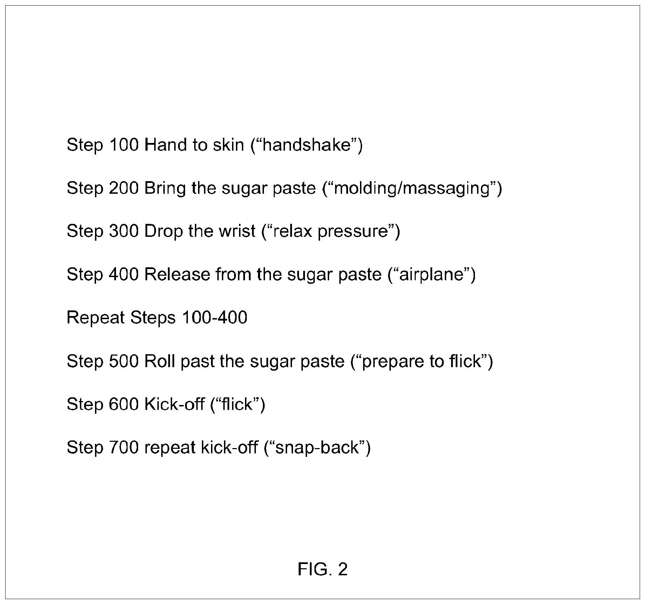

The following description is of an improved sugaring technique which can be used in a stand- alone fashion, but which has enhanced results as part of an overall skin conditioning program. This description is directed at an instructor who is teaching skin care professionals how to use the technique in salons. The technique may also be used at home. FIG. 2 is a summary of a 6 step improved sugaring technique.

### Before You Start - confirming hand position

Standing across from each student at the massage table, the instructor shakes hands with one student at a time and asks them to release from the handshake by dropping the hand to the table. The instructor observes how they place their hand when it reaches the table, and repeats this action until their hand is in the correct handshake position as it reaches the table. The students observe the instructor's hand as it reaches the table so they can mimic the proper hand position. This is to establish the correct hand position to begin treatment and the correct hand position to "flick" (remove) the sugar off the skin.

### Step 100 Hand to Skin ( "handshake position")

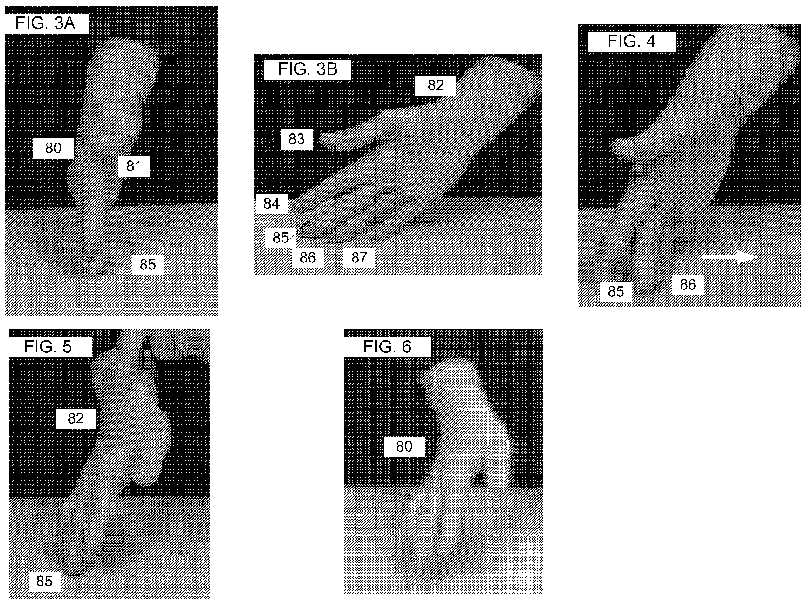

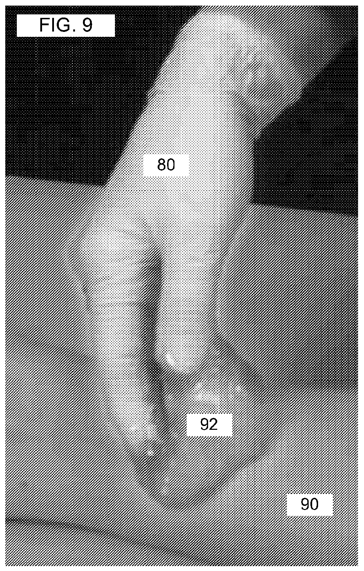

FIG. 3A is a front view illustration of a hand **80** and wrist **82** in a "handshake position" of Step **100** of the flow chart of FIG. 2. FIG. 3B is a side view showing the finger and wrist alignment, and finger separation of FIG. 3A where the middle finger **85**, ring finger **86**, and little finger **87** are applying equal pressure. FIGs 3-8 show hand positions without sugar paste. FIGs. 9-17 show the hand position with sugar paste **92** applied to a portion of a leg **90.**

In FIG. 3A, the index finger **84** and thumb **83** are not applying pressure. In Figs. 9-17, the index finger and thumb are used to help contain the sugar paste.

At step **100**, the technician places the sugar paste on the client. The sugar paste is placed on
the client's skin with the technician's sugaring hand. The sugar paste is placed on the client's skin, and not in the palm of the technician's hand. The sugar paste is confined to the inside of the technician's fingers and is not present behind the technician's fingers. As shown in FIG. 5, the hand is rotated slightly from vertical while the middle finger, ring finger, and little finger are aligned as in the handshake orientation. A controlled and moderate pressure of the technician's fingers **85**, **86**, and **87** and hand is maintained on the client's skin. In this example, the index finger **84** is not used to apply pressure to the skin. In other examples, the index finger is used to apply equal pressure as the middle finger **85**, ring finger **86**, and little finger **87.** The thumb **83** of the technician's sugaring hand is used to keep the sugar paste rolling onto the client's skin so that the sugar paste does not move to the technician's palm **81.**

FIG. 9 is a front view illustration of a hand **80** and wrist in the "handshake position" of Step **100** of the flow chart of FIG. 2 with sugar paste **92.**

If this step is performed correctly, then there is good penetration and seeping of the sugar paste into the follicles; and the technician is prepared to make an effective and comfortable application of the paste. Training gloves are useful in teaching the student to set and maintain proper hand and finger orientation in the sugaring process.

### Step 200 Bring the Sugar Paste ("molding/massage")

FIG. 4 is a front view showing the finger and wrist alignment for a sugar paste "molding" of Step **200** of the flow chart of FIG. 2.

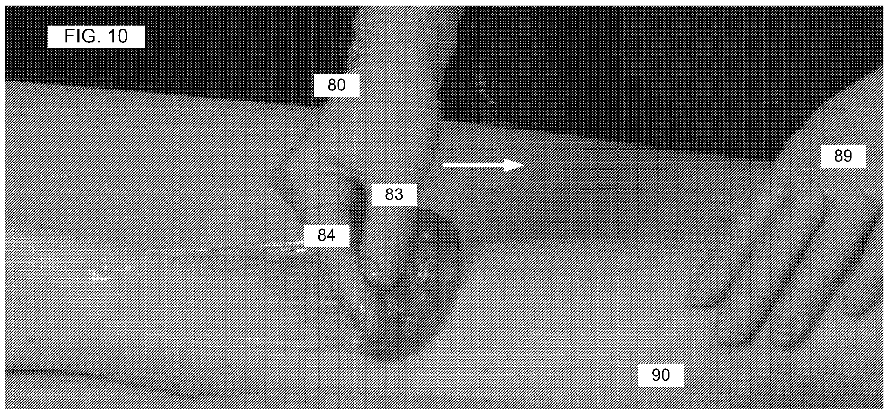

FIG. 10 is a front view showing the finger and wrist alignment and sugar paste **92** of the sugar paste "molding" of Step **200** of the flow chart of FIG. 2. In this example, the technician's second hand **89** is used to tighten the skin on the leg.

At step **200,** the technician maintains a hand position similar to Step 1, with the exception that the technician needs to control the wrist in order to bring the sugar forward. The thumb **83** of the sugaring hand is used to keep the sugar paste **92** off the palm and down on the skin and to keep the sugar paste rolling onto the skin as the technician applies the sugar paste. An even pressure is maintained on all fingertips being used to sugar, and the even pressure helps to keep a solid control of the wrist. The even pressure and wrist control facilitates an even application of the paste. The technician should spread the fingers of the application hand for smoother equal application whilst keeping the fingers in a parallel line to each other. If the fingers are kept too tightly close together, then it will affect the application, the time it takes to complete the treatment, especially of legs and arms, and the comfort of the application.

### Step 300 Drop Your Wrist ("relax/no pressure")

FIG. 5 is a front view showing the finger and wrist alignment of the "relax" wrist dropping of Step **300** of the flow chart of FIG. 2.

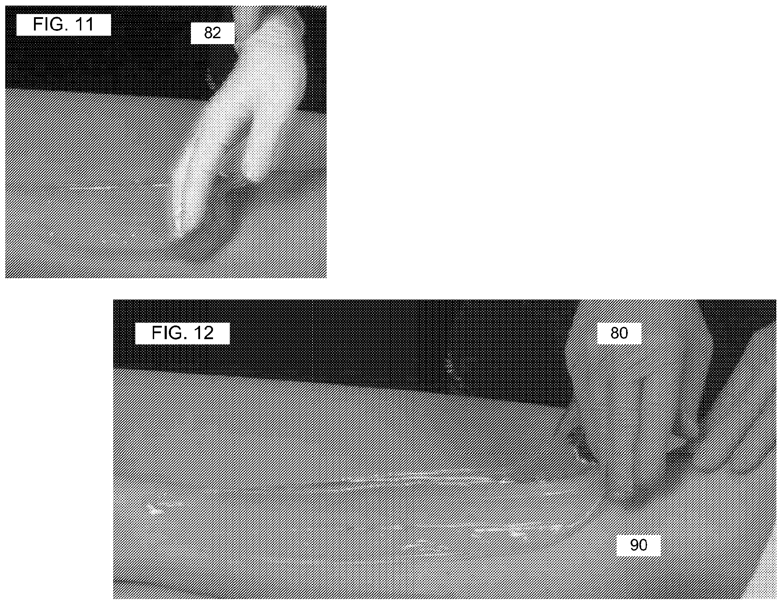

FIG. 11 is a front view showing the finger and wrist alignment and sugar paste of the "relax" wrist dropping of Step **300** of the flow chart of FIG. 2.

At step **300,** the technician immediately stops the application and molding of the sugar paste and prepares for steps **400,** or for steps **500** and **600.** As described below, steps **100-400** will typically be repeated once or twice before steps **500** and **600** are executed. If this step is performed incorrectly, then the client may experience discomfort or pain during the release; or may experience discomfort or pain and broken hair when "flicking".

### Step 400 Release from Sugar Paste ("airplane")

FIG. 6 is a front view showing the finger and wrist alignment of the "airplane" release of Step **400** of the flow chart of FIG. 2.

FIG. 12 is a front view showing the finger and wrist alignment and sugar paste of the "airplane" release of Step **400** of the flow chart of FIG. 2.

At step **400**, the technician releases the hand from the sugar paste without lifting the skin by executing an "airplane take-off" action - NOT a vertical "helicopter" action. The technician is then properly positioned to repeat steps **100** and **200.** If this step is performed incorrectly, then the technician can lift the client's skin or pull the client's hair and cause pain during release. The airplane take-off analogy provides a reminder to the technician to execute a release with the application hand at an acute angle to the skin without abruptly cutting away from the sugar paste.

### Repeat of steps 100-400

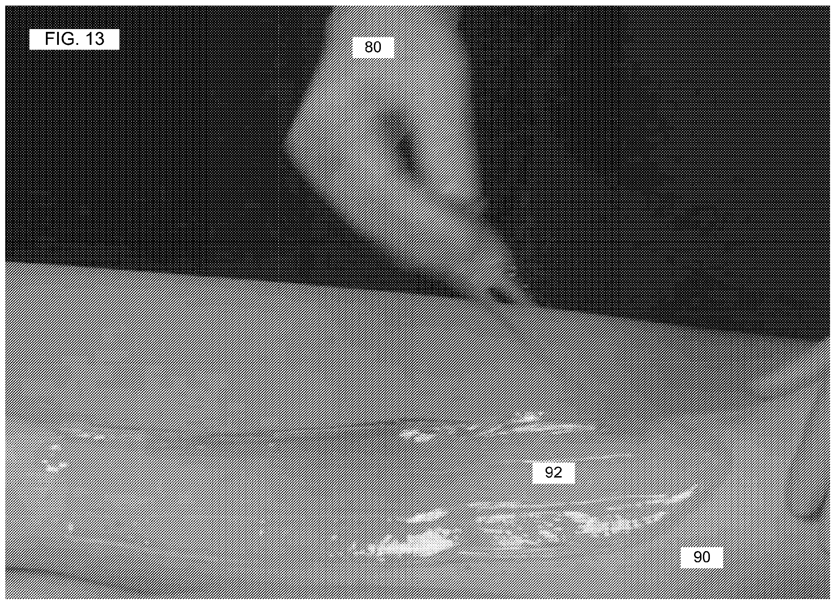

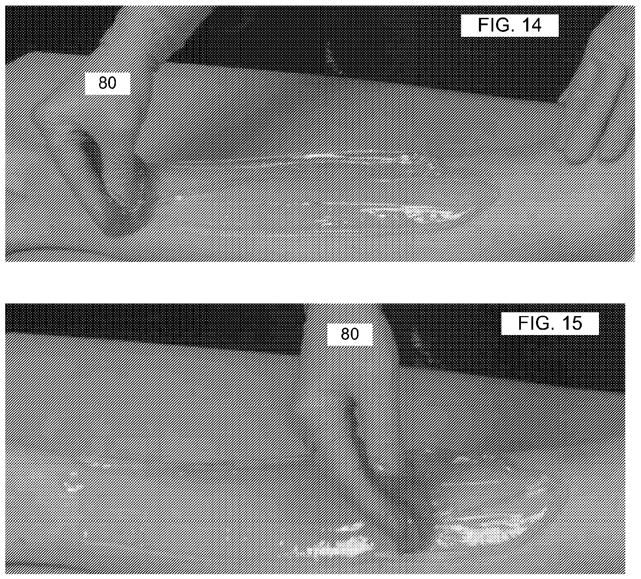

It is generally desirable to repeat the "molding" process once or twice over a region of skin before flicking off the sugar paste. This repeated molding helps to work the sugar into the follicles for better hair removal. FIGs 14-16 illustrate a repeat of steps **100-200.** FIG. 14 is a front view showing the return to a handshake position following the "kick off' of FIG. 13. FIG. 15 is a front view showing the molding following the "kick off' and return to a handshake position of FIG. 14.

### Step 500 Roll Past Sugar Paste ("prepare to flick")

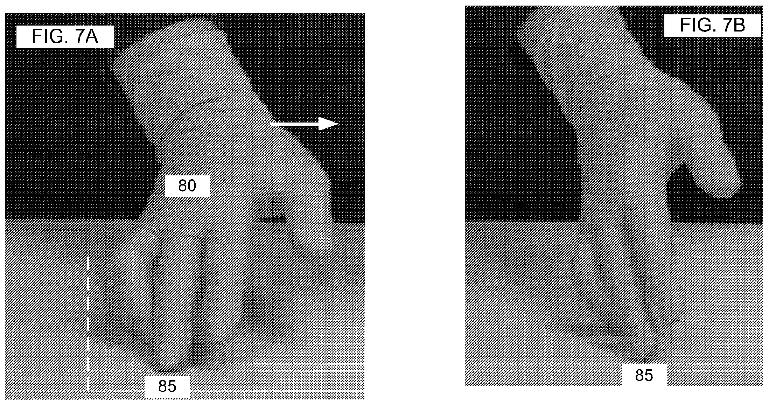

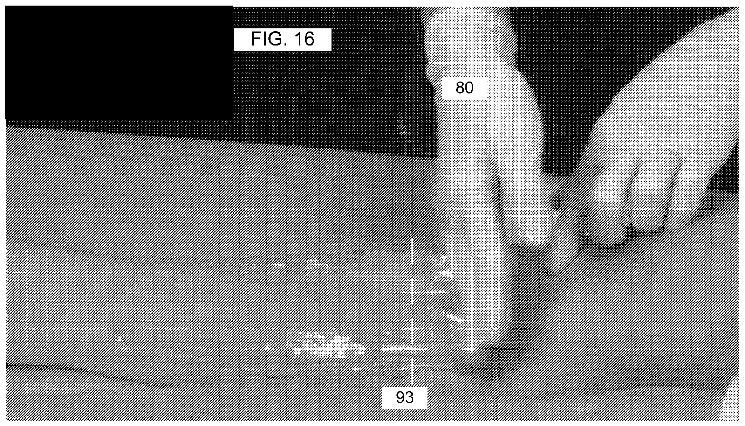

FIG. 7A is a front view showing the finger and wrist alignment of the "roll" of Step **500** of the flow chart of FIG. 2. FIG. 7B is a front view showing the finger and wrist alignment of the "prepare to flick" of Step **500** of the flow chart of FIG. 2. FIG. 16 is a front view showing the "roll" and return to a handshake position to "prepare to flick" following the "molding" steps of FIG. 14.

The sugar paste is not removed during this step. Step **500** should be performed correctly in order to prepare for step **600.** The purpose for performing step **500** is to allow the technician to stop with the pressure of step **200** after they have come to step **300**, and to "roll past" the applied sugar on the skin while reducing their contact pressure by 50% and continuing to roll on the applied sugar to get to the end of the applied sugar paste. The technician continues to apply equal pressure to all fingers on the application hand. The technician prepares for the "kick-off" (Step **600**) by connecting to the end of the sugar paste application. This is similar to removing a piece of tape, by gripping the very edge of the tape to remove it.

### Step 600 Kick-Off ("flick")

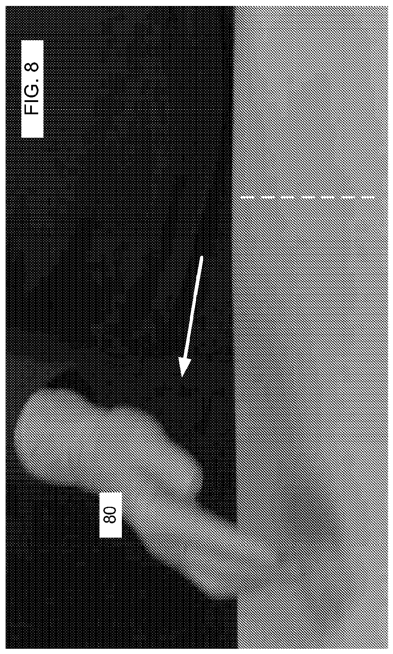

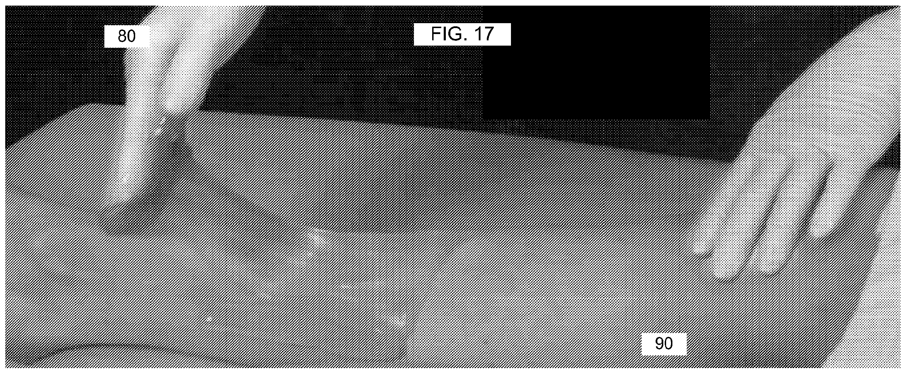

FIG. 8 is a front view showing the finger and wrist alignment of the "kick off" of Step **600** of the flow chart of FIG. 2. FIG. 17 is a front view showing a "kick off' following the "prepare to flick" of FIG. 16.

At step **600**, the technician maintains a comfortable relaxed wrist and application position for the "Kick command" while still moving onto the skin past the applied paste. If the technician sees the palm of the application hand, then the technician should not perform step **600**, but should attempt to reposition the hand more into the same relaxed position of step **300.** If that fails, the technician should start over at step **100.**

In the preferred technique, the technician's fingers remain in a parallel position with equal reduced pressure applied by each finger. A kick-back maneuver is executed without lifting the application hand off the sugar paste. The instructor preferably issues an audible or non-audible command to stress the word "KICK", and a command to stress the word "Handshake" whilst continuing to gently move on the skin and the final command of the word "OFF". A firm wrist action is used when performing the "OFF" voice command, with little effort from the technician's shoulder. The "OFF" command should immediately follow the "Handshake" command without the slightest hesitation. If this step is performed incorrectly, then the client may experience discomfort or pain from lifted skin or broken hair. The technician should stay connected to the sugar and skin when "kicking" and when going from a step **300** positioning of the hand.

### Second and Consecutive Kick-Off using "Snap-back" action ("the art of sugaring")

The technician should immediately snap-back the kicked-off (flicked-off) sugar paste to accumulate it on the client's skin in order to prevent the sugar paste from travelling on the client's skin and to effectively sugar each molded strip while performing professional sugaring treatments. Snap-back will allow the technician to control the sugar paste as it is repeatedly "flicked" off the skin and to prevent it from flying away from the technician's hand and onto the treatment table, the floor, or walls. Snap-back is an intricate part of the ongoing technique of the 6 Steps to perfect sugaring.

By deciding exactly where one wants to accumulate the "flicked sugar", the technician can perform step **600** and immediately return her sugaring hand connected to the "flicked sugar paste" to the exact predetermined spot on the skin. This will allow her to accumulate/gather the sugar paste in a small "pile", perform step **100** behind the sugar paste pile to collect it and continue to sugar that strip until it is completely off the skin.

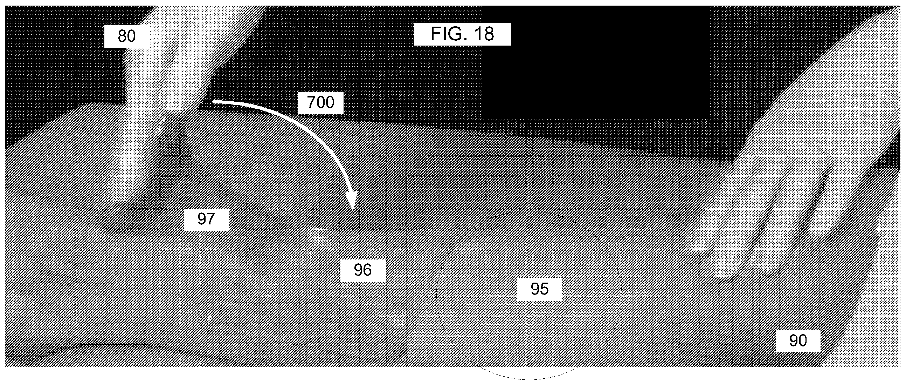

FIG. 18 is an illustration of a "snap back" following the "kick off" of FIG. 17. In this figure, the portion **97** of the sugar paste has just been flicked off of area **95** of the leg, and the technician is preparing to "snap back" that portion **97** to area **96** to repeat the "flick" for region **96.**

Predetermining where to return the sugaring hand after performing step **600** will help the technician to prevent "travelling on the skin" either in front or behind the applied sugar paste. This will prevent the technician from overworking an area, from getting stuck on the skin and help them to become quicker at the treatment without compromising efficiency.

After each "kick-off' the technician should immediately position the application hand
according to step **100** and immediately move to step **300.** Step **200** should be eliminated whenever the technician is ready to execute a series of kick-off steps. Step **500** (and then step **600**) is performed if there is a distance of sugar paste remaining on the skin; and step **600** is performed after step 300 if the sugar paste distance is minimal such as 2.5 to 5 cm (1 to 2 inches) maximum.

If these steps are performed incorrectly, then the client may experience discomfort or pain from lifted skin or broken hair.

Over performing Step **200** will overwork the sugar paste and likely melt it too much into the skin and get the technician stuck. The technician can cause some irritation from trying to Kick-Off sugar paste that is melted into the skin. This can also cause the technician to become too hot from frustration. If this melting occurs, the technician should simply relax, gently remove the application hand from the sugar paste using Step **400**, and continue to sugar in another area. When you leave the melted sugar paste for a few minutes, it will cool down and be easier to remove.

### Example - series of sugaring steps on client arm

The technician executes steps **100-600** at a first upper portion of the arm with the application hand.

After the application of the sugar paste on a strip of the upper arm and the initial kick-off, the technician brings the hand to the position of step **100** over a second portion of skin slightly lower on the upper arm, collects the sugar paste that was kicked-off by placing the hand just behind the collected paste, and executes steps **300,** step **500** and then step **600.** These steps are repeated until the applied strip of sugar paste is completely removed from the skin. (Step **200** is eliminated for several successive kick-off sequences, and step **500** is eliminated when there is less than 5 cm (2 inches) distance of paste remaining on the client's skin.)

After the first applied strip of sugar paste is removed and all the hair beneath the applied sugar paste is extracted, the technician brings the hand to the position of step **100** over another section of the upper arm portion of skin and executes steps **300, 400, 500** and step **600.** (Step **200** is eliminated for several successive kick-off sequences, and step **500** is eliminated when there is less than 5 cm (2 inches) distance of paste remaining on the client's skin.)

These steps are repeated until each section of the upper arm is completed and all hair is extracted.

After the upper arm is completed, the technician brings the hand to the position of step **100** over a portion of skin on the underneath part of the lower arm, executes steps **300**, **400**, **500** and step **600.** Afterwards, the same protocol in applying the steps is followed as with the upper arm. Once the underneath portion of the lower arm is completed and all hairs are extracted, the technician then moves to the top forearm. The hair in this portion of the arms grows sideways. The technician follows the natural direction of growth by positioning the hand to step **100** and executes step **200**, **300**, **400**, **500** and step **600** respectively from the outside of the forearm to the inside of the forearm.

After the final kick-off of the first strip applied to the lower top forearm, the technician brings the hand to the position of step **100** over a new portion of the skin on the forearm, and executes steps **100-600.**

The technician should inspect the direction of the hair growth prior to beginning treatments using the 6 Steps. The Kennedy Theory^{®} executes the extraction of hair in the natural direction of hair growth, and this requires that Steps **100** and **200** be executed against the natural direction for better penetration of the sugar paste and an even gentler execution of step **600.**

Each Step has magic to its performance, and relies on the other steps for perfect technique performance.

### Summary

With some patience, the technician can gain experience and master the magic of Alexandria Professional^{®} body sugaring hair removal and skin conditioning treatments.

Step **100** provides the technician full control of the sugar paste, establishes her positioning on the skin and the correct pressure. Step **200** allows the technician to mold/apply the sugar paste without causing pain or discomfort to the client and to seep the sugar paste into the follicle. Step **300** allows the technician to prepare for either the release of the hand from the sugar and skin, or the removal of the sugar paste without causing pain or discomfort to the client. Step **400** allows the technician to return to continual Step 200 molding process without causing pain or discomfort. Step **500** allows the technician to prepare for the removal of the sugar paste without causing pain or discomfort.

Step **600** allows the technician to effectively remove the sugar paste and extract the hair and to perform the "snap-back" without causing pain or discomfort. After each "kick-off' the technician should immediately position the application hand according to step **100** and immediately move to step **300.**

### Variations

When you are ready to prepare for the Kick-off, you no longer need to perform step **200.** If you continue with step **200** every time you perform step **600**, then you will overwork the sugar paste and the skin and cause the sugar to melt too much into the skin.

The purpose of step **400** is to correctly release from the sugar paste on the skin without causing discomfort when you are going back to repeat the molding step **200.**

You only need to use step **500** when there is enough sugar paste molded onto the skin for you to "roll past" in preparation for the Kick-Off. If there is only a small piece of sugar paste such as the very last Kick-Off, then you do not use step **500**, but perform steps **100**, **300**, and **600.**

### Hand and Finger Position

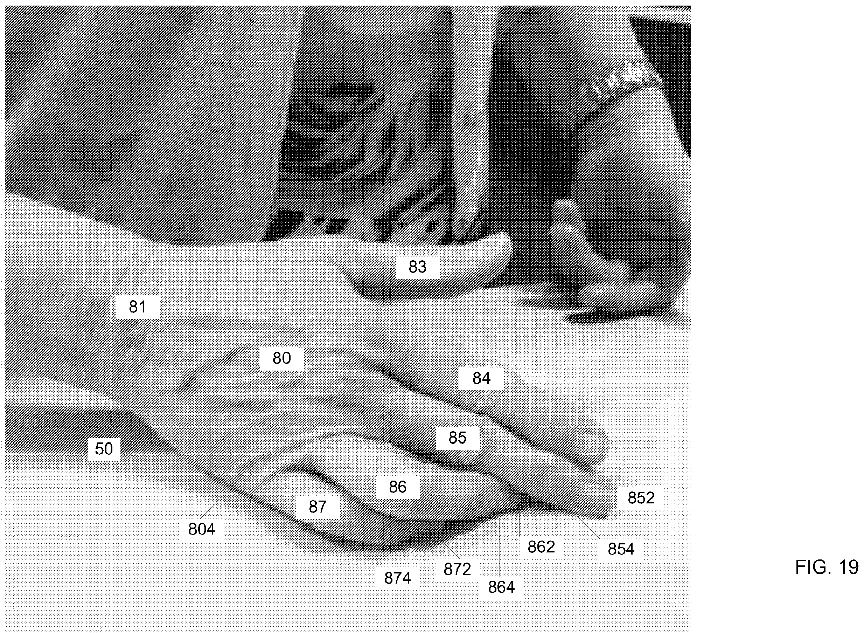

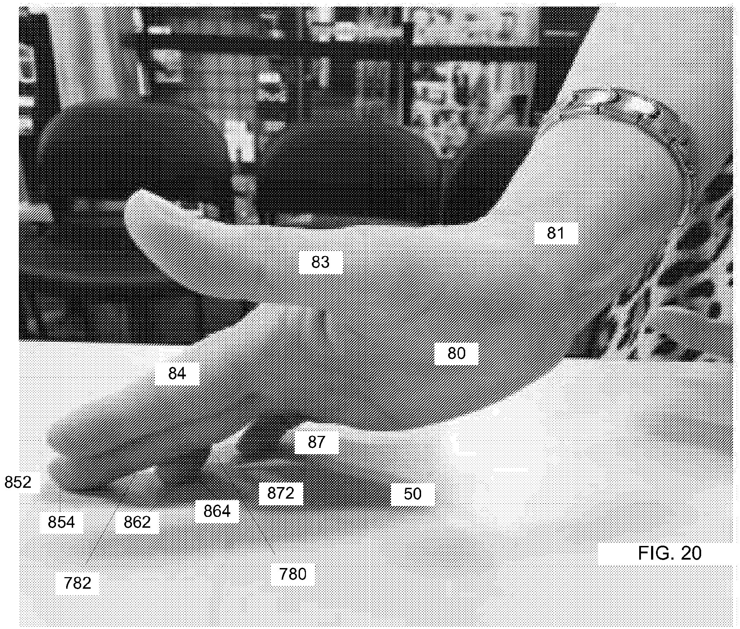

FIG. 19 is a right-side perspective view of a right hand **80** held in a first orientation for applying a sugaring composition. In this case the right hand **80** is held aligned with the wrist **82.** FIG. 19 shows the middle finger, ring finger, and little finger close together with fingertips aligned. In the various steps, such as shown in FIG. 20, the technician may separate the fingertips while maintaining the bending of the little finger and ring finger.

The index finger **84** and the middle finger **85** are extended outwardly from the hand, and the right portion **854** of the tip **852** of the middle finger is resting lightly on the surface **50.** In this example, the index finger **84** and the middle finger **85** are straight, and angled toward the palm at an angle of approximately 20-30 degrees so that the tip **852** of the middle finger is comfortably resting on the surface **50.**

The ring finger **86** and little finger **87** are each bent at the first knuckle, second knuckle, and third knuckle so that the right portion **874** of the tip **872** of the little finger, the right portion **864** of the tip **862** of the ring finger, and right portion **874** of the tip **872** of the middle finger are aligned and resting comfortably on the surface.

This alignment of the tips of the bent little finger, bent ring finger, and angled middle finger creates a dam for containing the sugar paste composition as the hand is drawn in a backward direction along the surface to initially apply the composition. A finger alignment training device such as a training glove or form assists the technician in learning and practicing this desired finger orientation.

In this orientation, the distal portion of the right-side base of the hand **804** is maintained in contact with the surface. This contact further contains the sugar composition. In sugaring application, the surface **50** would be a portion of a human body such as a leg, arm, or torso.

FIG. 20 is a rear left side perspective view of a right hand **80** held in a second orientation for applying a sugaring composition. In this case the fingers and thumb are maintained in the orientation described in FIG. 19 with the right-side tips of the little finger, ring finger, and middle finger in contact with the surface. The distal portion of the right-side base of the hand is raised above the surface by about 1.3 cm (one half inch).

As the right-side base of the hand is raised above the surface, a first separation **876** is created between the tip of the little finger and the tip of the ring finger; and a second separation **866** is created between the tip of the ring finger and the tip of the middle finger.

This alignment of the tips of the bent little finger, bent ring finger, and angled middle finger facilitates spreading the sugar paste composition as the hand is drawn in a backward direction along the surface to initially apply the composition.

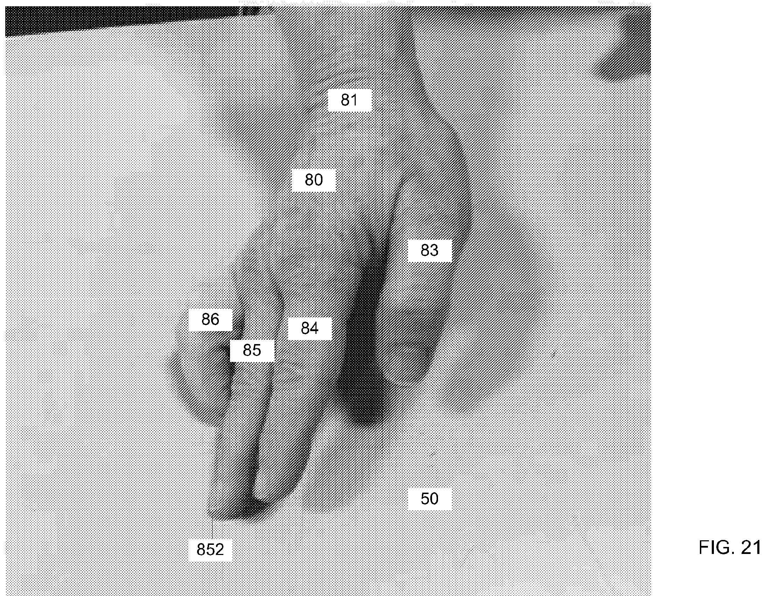

FIG. 21 is a top perspective view of a right hand **80** held in an orientation for applying a sugaring composition.

Establishing and maintaining the desired finger positions is important for a proper sugaring technique. Failure to maintain the proper finger positions can cause discomfort and can reduce the efficiency of the process. It is difficult to teach and to learn these finger positions. One way to improve the learning process is to provide a glove or other training device that assists the technician in learning and maintaining the proper finger positions. A finger alignment training device preferably permits the technician to adjust the spacing between fingertips while maintaining a desired curve and alignment of the fingers.

### EXAMPLE- training glove with dorsal finger inserts

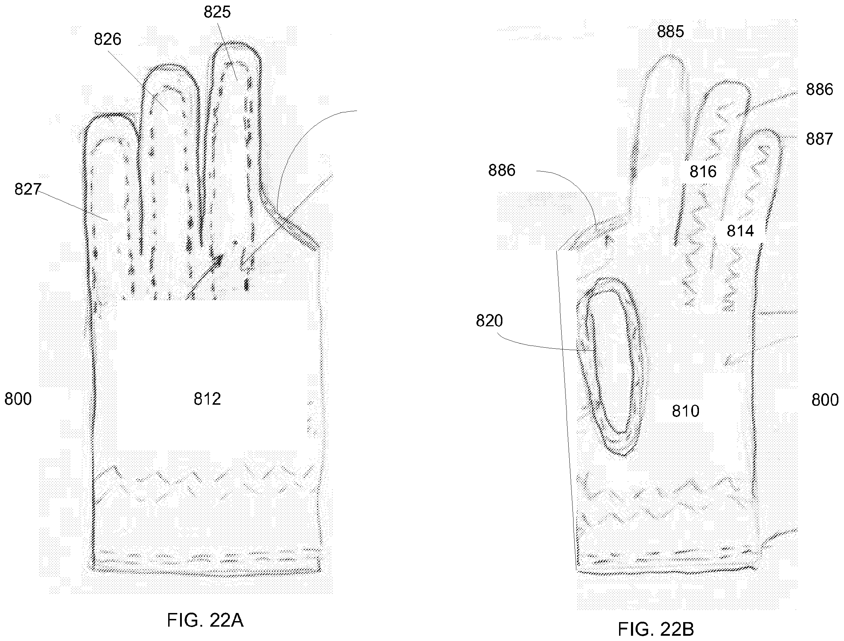

FIG. 22A is a top view of a training glove **800** embodiment showing the back side of a glove 812. FIG. 22B is a palm-side **810** view of the training glove embodiment of FIG. 22A.

In this example, the thumb and index finger are not constrained by the glove. The thumb is inserted through opening **820**, and the index finger is exposed through opening **886.** By remaining unconstrained, the thumb and index finger positions can be easily changed, such as moving to an orientation that prevents the sugar paste from accumulating on the palm.

The glove includes a little finger sleeve **887**, a ring finger sleeve **886**, and a middle finger sleeve **885.** In some examples, these finger portions are completely enclosed. In other examples, the finger tips are exposed by providing openings in the tops of the sleeves.

In this example, the back or dorsal side of the glove includes pocket **825** for insertion of a middle finger alignment guide, pocket **826** for insertion of a ring finger alignment guide, and pocket **827** for insertion of a little finger alignment guide. These alignment guides keep the middle finger, the ring finger, and the little finger in a desired curvature as shown in FIG. 20.

In this example, the middle finger alignment guide, the ring finger alignment guide, and the little finger alignment guide are inserted into the respective pockets, and the trainee puts the glove on the hand that will apply the sugar. A sugaring glove may be worn over the training glove. An instructor assists in bending the trainee's middle finger, the ring finger, and little finger into a handshake position so that when the hand is positioned on the client's skin, the middle fingertip, ring fingertip, and little fingertip are aligned in a substantially straight line where the fingertips apply a gentle pressure to the skin. In this example, the alignment guides are bent when the fingers are bent so that the guides can maintain the desired bends at the first, second, and third knuckles of the ring finger and little finger. The middle finger typically is nearly straight and has substantially less curvature than the ring finger and middle finger.

In other examples, the middle finger alignment guide, the ring finger alignment guide, and the little finger alignment guide may be bent prior to the trainee putting on the glove.

### EXAMPLE- training glove with palmar finger inserts

In other examples, a middle finger alignment guide, ring finger alignment guide, and little finger alignment guide may be inserted into pockets on the palmar side of the fingers.

### EXAMPLE- glove with both palmar and dorsal finger inserts

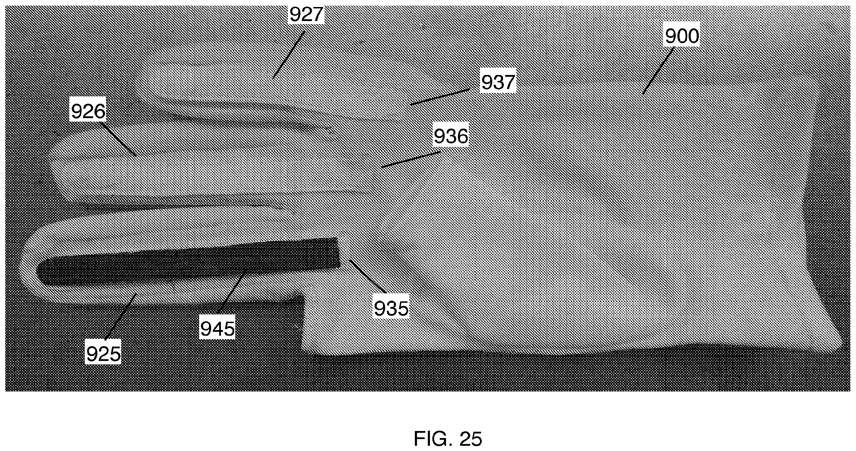

FIGs. 25-29 show an example training glove with both palmar and dorsal finger alignment guides. FIG. 25 is a back view of a training glove **900** showing a little finger pocket **927** with bottom opening **937**; a ring finger pocket **926** with bottom opening **936**; and a middle finger pocket **925** with bottom opening **935.** An unbent middle finger alignment guide **945** is shown on top of the middle finger slot. In this example, the finger alignment guides are provided as a flexible metal or plastic that can be bent to a desired shape after insertion into the respective sleeves.

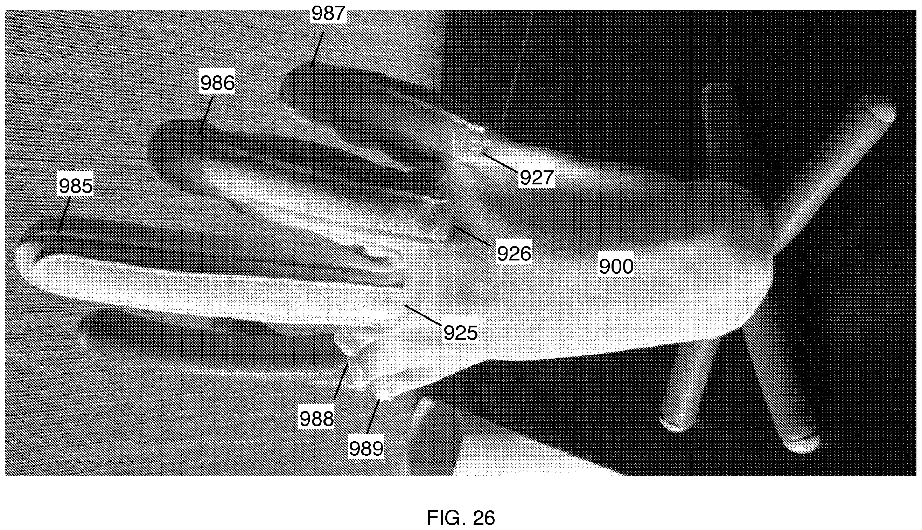

FIG. 26 is a back view of the training glove **900** on a stand and showing an open index finger portion **988**, an open thumb portion **989**, a little finger sleeve **987** with pocket **927**; a ring finger sleeve **986** with pocket **926**; and a middle finger sleeve **985** with pocket **925.** In this example, the glove is stretchable, comfortable, and light weight such as a golf glove, and has a back portion made of smooth face and front mesh portion. In other examples, other materials may be used.

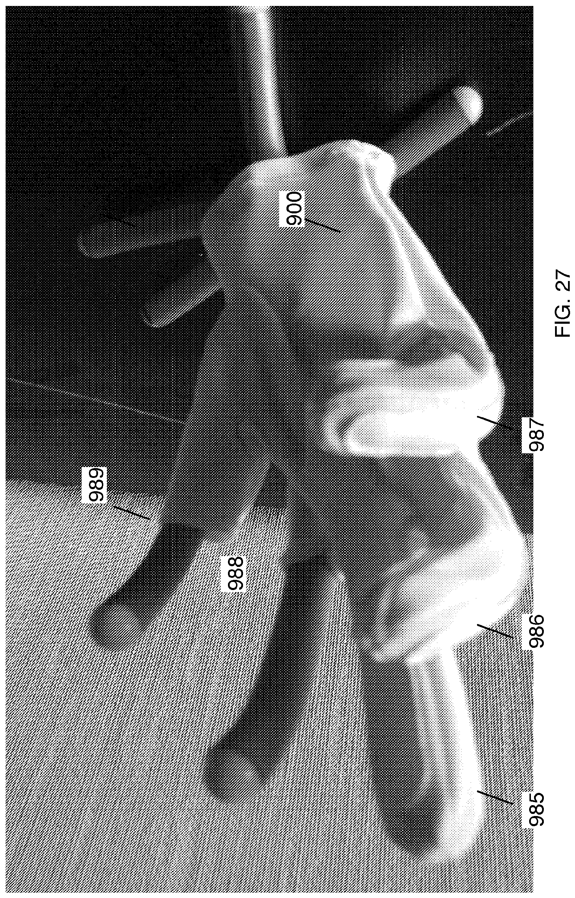

FIG. 27 is a side view of the training glove **900** on a stand and showing bent finger positions for the little finger portion **987**, the ring finger portion **986,** and the middle finger portion **985.** In this example, before the technician puts the glove on, an unbent middle finger alignment guide **945** is inserted into the middle finger sleeve **925**, an unbent ring finger alignment guide **946** is inserted into the ring finger sleeve **926**, and an unbent little finger alignment guide **947** is inserted into the little finger sleeve. Once the glove is put on, the alignment guides are bent into desired orientations.

In other examples, the alignment guides are bent prior to putting the glove on.

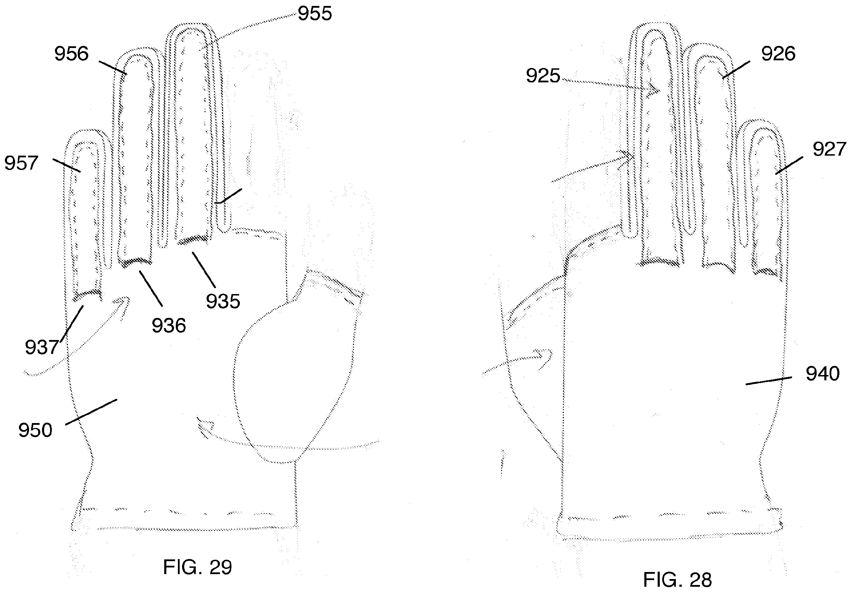

FIG. 28 is a back view showing the stitching of the back **940** of glove **900** showing dorsal pockets 927, 926, and 925 for a little finger, ring finger, and middle finger, respectively.

FIG. 29 is a front view of glove **900** showing palmar pockets **957**, **956,** and **955** for a little finger, ring finger, and middle finger, respectively. Slight variations in finger and hand positioning can have a pronounced effect on sugaring comfort and efficiency, and testing has shown that a combination of palmar and dorsal alignment guides are more effective at holding desired finger orientations than single dorsal or palmar finger alignment guides.

In this example, finger alignment guides are provided for the middle finger as well as the ring finger and little finger. Although the middle finger is preferable kept substantially straight, it is helpful to provide a finger alignment guide so that the technician can maintain correct alignment.

In other examples, the training device may include only finger alignment guides for the ring finger and little finger.

In this example, the cutouts or openings are provided for the thumb and forefinger. In other examples, a training glove may include sleeves for the index finger, thumb, or both. Fingertip holes may be provided at top of the sleeves, such as to accommodate long fingernails.

### EXAMPLE- glove with detachable alignment guides

In the examples above, the finger alignment guides are provided in pockets provided in the finger sleeves. In other examples, the finger alignment training device is a glove with detachable finger alignment guides, where the guides may be removable and attached to the finger sleeves with hook and loop fastener, double sided tape, snap fit, or other means.

### EXAMPLE- glove with affixed alignment guides

In another example, the finger alignment training device is a glove where finger alignment guides are affixed to the glove.

### EXAMPLE- multi-finger form

In other examples, finger alignment guides are part of a single form or template rather than a glove. The form or template may be performed to a desired curvature, and positioned on the dorsal or palmar side of the fingers and held in place by an elastic strap or other means. In other examples, a flexible form is provided and bent into a desired shape as the fingers are bent.

### EXAMPLE- dual multi-finger forms

In other examples, the finger alignment training device is provided as one or more form where finger alignment guides are part of a form that sandwiches at least the ring finger and little finger between a palmar portion and a distal portion. In one example, a flexible form is provided and bent into a desired shape as the fingers are bent. In another example the palmar portion and distal portion are pre-formed and are fastened together after being positioned on the fingers.

In other examples, a single insert form is provided to position all three fingers- the middle finger, the ring finger, and the little finger.

One advantage to the single finger inserts, or to the multiple finger form, is the ability to help double-jointed technicians establish proper finger positioning. In this example, the desired position of the little finger and ring finger requires bending the ring finger or the little finger at the first and second knuckles, and double-jointed persons have difficulty maintaining a desired bend at both knuckles. The guides help to hold the desired bent-finger positions.

In addition to establishing the desired finger bending, the individual finger inserts or the single insert form position the fingers so that the outside tips of the middle finger, ring finger, and little finger are properly aligned. This alignment helps to apply an appropriate pressure to the skin in a sugaring application.

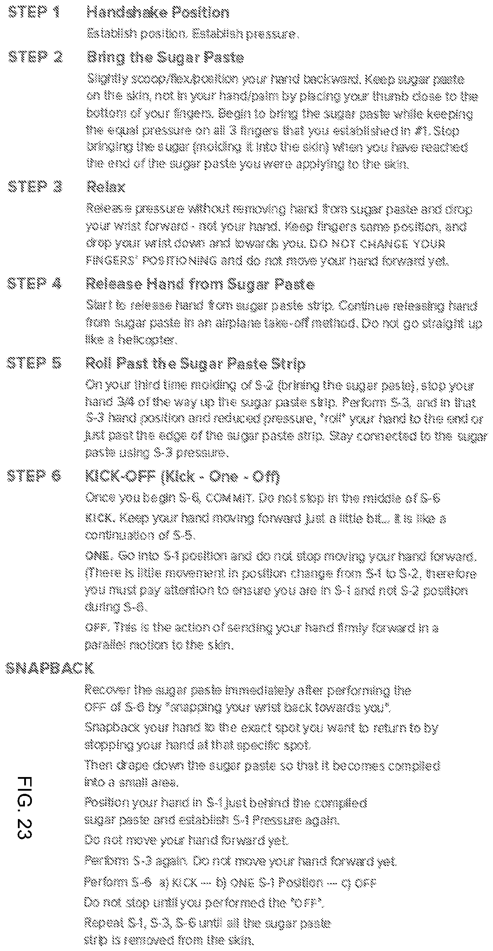

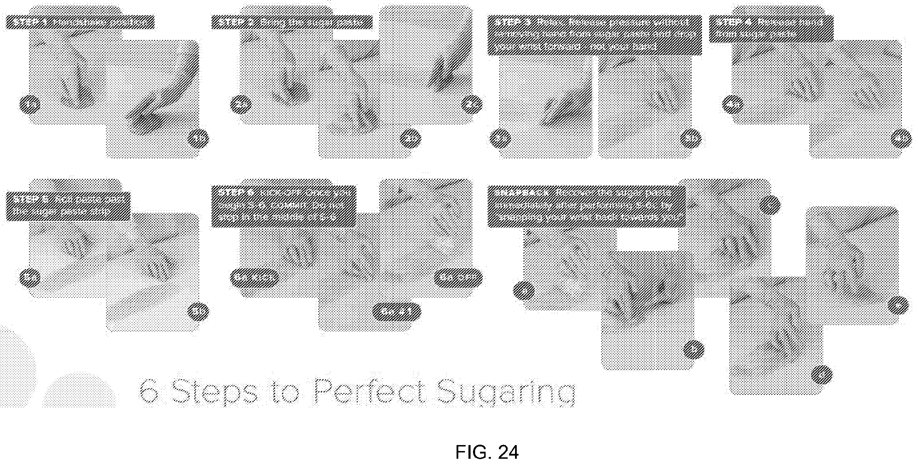

FIG. 23 summarizes the 6 Steps to Perfect Sugaring. FIG. 24 illustrates the 6 Steps to Perfect Sugaring. In one example, a glove with finger inserts is used to establish and maintain the desired finger positions for the middle finger, ring finger, and little finger throughout the 6 steps.

The glove may be used for an initial training period, until the technician has established the habit of proper finger positioning. The glove may also be used on a routine basis after initial training, or periodically to confirm proper sugaring technique.

While exemplary embodiments of the invention have been described, it should be apparent that modifications and variations thereto are possible, all of which fall within the true scope of the invention. The above description and drawings are illustrative of modifications that can be made without departing from the present invention, the scope of which is to be limited only by the following claims. Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications are intended to fall within the scope of the invention as claimed.

## Claims

1. A finger alignment device (800; 900) for finger positioning of a little finger (87) and little fingertip (872), ring finger (86) and ring fingertip (862), and middle finger (85) and middle fingertip (852), the device comprising:
a little finger portion (887; 987) comprising a first bent little finger alignment guide that contours the bending of a little finger at a first knuckle, a second knuckle, and a third knuckle;
a ring finger portion (886; 986) comprising a first bent ring finger alignment guide that contours the bending of a ring finger at a first knuckle, a second knuckle, and a third knuckle; and
a middle finger portion (885; 985) comprising a first middle finger alignment guide (945);
**characterised in that** the device is a finger alignment training device (800; 900) for teaching proper finger positioning while performing a cosmetic sugaring hair removal procedure, and **in that** the bent little finger alignment guide, the bent ring finger alignment guide, and the middle finger alignment guide are configured to position the little fingertip (872), the ring fingertip (862), and the middle fingertip (852) along a line as the little fingertip, ring fingertip, and middle fingertip are spaced apart.

2. The finger alignment training device of claim 1 wherein the training device is a training glove comprising
a little finger sleeve (887; 987) having a dorsal portion and a palmar portion,
a ring finger sleeve (886; 986) having a dorsal portion and a palmar portion, and
a middle finger sleeve (885; 985) having a dorsal portion and a palmar portion.

3. The finger alignment training device of claim 2 wherein
the first bent little finger alignment guide, the first bent ring finger alignment guide, and the first middle finger alignment guide (945) are separate glove finger inserts,
a first little finger pocket (827; 927) is provided on the dorsal portion of the little finger sleeve (887; 987) and configured to accept the first bent little finger alignment guide,
a first ring finger pocket (826; 926) is provided on the dorsal portion of the ring finger sleeve (886; 986) and configured to accept the first bent ring finger alignment guide, and
a first middle finger pocket (825; 925) is provided on the dorsal portion of the middle finger sleeve (885; 985) and configured to accept the first bent middle finger alignment guide.

4. The finger alignment training device of claim 2 wherein
the first bent little finger alignment guide, the first bent ring finger alignment guide, and the first middle finger alignment guide (945) are separate glove finger inserts,
a first little finger pocket (827; 927) is provided on the palmar portion of the little finger sleeve (887; 987) and configured to accept the first bent little finger alignment guide,
a first ring finger pocket (826; 926) is provided on the dorsal portion of the ring finger sleeve (886; 986) and configured to accept the first bent ring finger alignment guide, and
a first middle finger pocket (825; 925) is provided on the dorsal portion of the middle finger sleeve (885; 985) and configured to accept the first bent middle finger alignment guide.

5. The training device of claim 2 wherein
the little finger sleeve (887; 987) has an opening configured to expose the tip of a little finger (87),
the ring finger sleeve (886; 986) has an opening configured to expose the tip of a ring finger (86), and
the middle finger sleeve (885; 985) has an opening configured to expose the tip of a middle finger (85).

6. The training device of claim 2 further comprises
a thumb cutout (820; 989) and an index finger cutout (886; 988).

7. The training device of claim 3 further comprising
a second bent little finger alignment guide and a second bent ring finger alignment guide are each inserted into pockets provided on the palmar side of the little finger portion and the ring finger portion, respectively of the training glove.

8. The training device of claim 2 further comprising
a little finger sleeve (887; 987) such that the first bent little finger alignment guide is removably attached to the dorsal side of the little finger sleeve;
a ring finger sleeve (886; 986) such that the first bent ring finger alignment guide is removably attached to the dorsal side of the ring finger sleeve; and
a middle finger sleeve (885; 985) such that the first middle finger alignment guide is removably attached to the dorsal side of the middle finger sleeve.

9. The training device of claim 2 further comprising
a little finger sleeve such that the first bent little finger alignment guide is removably attached to the palmar side of the little finger sleeve;
a ring finger sleeve such that the first bent ring finger alignment guide is removably attached to the palmar side of the ring finger sleeve; and
a middle finger sleeve such that the first middle finger alignment guide is removably attached to the palmar side of the middle finger sleeve.

10. The training device of claim 2 further comprising a little finger sleeve such that
the first bent little finger alignment guide is removably attached to the dorsal side of the little finger sleeve, and
a second bent little finger alignment guide is removably attached to the palmar side of the little finger sleeve; and
a ring finger sleeve such that the first bent ring finger alignment guide is removably attached to the dorsal side of the ring finger sleeve, and
a second bent little finger alignment guide is removably attached to the palmar side of the little finger sleeve.

11. The training device of claim 2 further comprising
a little finger sleeve (887; 987) such that the first bent little finger alignment guide is affixed to the little finger sleeve;
a ring finger sleeve (886; 986) such that the first bent ring finger alignment guide is affixed to the ring finger sleeve; and
a middle finger sleeve (885; 985) such that the first middle finger alignment guide is affixed to the middle finger sleeve.

12. The training device of claim 1 wherein
the first bent little finger alignment guide, the first bent ring finger alignment guide, and the first middle finger alignment guide are provided as a first form.

13. The training device of claim 12 wherein
the first form is attached to the dorsal side of at least one of the little finger, ring finger, or middle finger.

14. The training device of claim 12 wherein
the first form is attached to the palmar side of at least one of the little finger, ring finger, or middle finger.

15. The training device of claim 13 further comprising
a second form comprising
a second bent little finger alignment guide that contours the bending of a little finger at a first knuckle, a second knuckle, and a third knuckle, and
a second bent ring finger alignment guide that contours the bending of a ring finger at a first knuckle, a second knuckle, and a third knuckle,
such that the second form is attached to the palmar side of at least one of the little finger or ring finger.

## Patentansprüche

1. Eine Fingerausrichtungsvorrichtung (800; 900) für die Fingerpositionierung eines kleinen Fingers (87) und einer Spitze des kleinen Fingers (872), eines Ringfingers (86) und einer Ringfingerspitze (862) und eines Mittelfingers (85) und einer Mittelfingerspitze (852), wobei die Vorrichtung umfasst:
einen abschnitt des kleinen Fingers (887; 987), der eine erste Ausrichtungsführung für den gebogenen kleinen Finger aufweist, die die Biegung eines kleinen Fingers an einem ersten Knöchel, einem zweiten Knöchel und einem dritten Knöchel konturiert;
einen Ringfingerabschnitt (886; 986) mit einer ersten Ausrichtungsführung für den gebogenen Ringfinger, die die Biegung eines Ringfingers an einem ersten Knöchel, einem zweiten Knöchel und einem dritten Knöchel konturiert; und
einen Mittelfingerabschnitt (885; 985) die eine erste Mittelfingerausrichtungsführung aufweist (945);
**dadurch gekennzeichnet, dass** die Vorrichtung eine Vorrichtung zum Trainieren der Fingerausrichtung (800; 900) zum Erlernen der richtigen Positionierung der Finger bei der Durchführung einer kosmetischen Sugaring-Haarentfernungsprozedurist, und dass die Ausrichtungsführung für den gebogenen kleinen Finger, die Ausrichtungsführung für den gebogenen Ringfinger und die Ausrichtungsführung für den Mittelfinger so gestaltet sind, dass sie die Spitze des kleinen Fingers (872), die Ringfingerspitze (862) und die Mittelfingerspitze (852) entlang einer Linie positionieren, wobei die Spitze des kleinen Fingers, die Ringfingerspitze und die Mittelfingerspitzevoneinander beabstandet sind.

2. Vorrichtung zum Trainieren der Fingerausrichtung nach Anspruch 1, wobei die Trainingsvorrichtung ein Trainingshandschuh ist, der Folgendes umfasst
eine Hülse für den kleinen Finger (887; 987) mit einem dorsalen und einem palmaren Abschnitt,
eine Ringfingerhülse (886; 986) mit einem dorsalen und einem palmaren Abschnitt und eine Mittelfingerhülse (885; 985) mit einem dorsalen und einem palmaren Abschnitt.

3. Vorrichtung zum Trainieren der Fingerausrichtung nach Anspruch 2, wobei
die erste Ausrichtungsführung für den gebogenen kleinen Finger, die erste Ausrichtungsführung für den gebogenen Ringfinger und die erste Ausrichtungsführung für den Mittelfinger (945) sind separate Handschuhfingereinsätze,
eine erste Tasche für den kleinen Finger (827; 927) ist am dorsalen Abschnitt der Hülse für den kleinen Finger (887; 987) vorgesehen und so gestaltet, dass sie die erste Ausrichtungsführung für den gebogenen kleinen Finger aufnimmt,
eine erste Tasche für den Ringfinger (826; 926) ist am dorsalen Abschnitt der Hülse für den Ringfinger (886; 986) vorgesehen und so gestaltet, dass sie die erste Ausrichtungsführung für den gebogenen Ringfinger aufnimmt,
eine erste Tasche für den Mittelfinger (825; 925) ist am dorsalen Abschnitt der Hülse für den Mittelfinger (885; 985) vorgesehen und so gestaltet, dass sie die erste Ausrichtungsführung für den gebogenen Mittelfinger aufnimmt.

4. Vorrichtung zum Trainieren der Fingerausrichtung nach Anspruch 2, wobei
die erste Ausrichtungsführung für den gebogenen kleinen Finger, die erste Ausrichtungsführung für den gebogenen Ringfinger und die erste Ausrichtungsführung für den gebogenen Mittelfinger (945) sind separate Handschuhfingereinsätze,
eine Tasche für den ersten kleinen Finger (827; 927) ist am palmaren Abschnitt der Hülse für den kleinen Finger (887; 987) vorgesehen und so gestaltet, dass sie die erste Ausrichtungsführung für den gebogenen kleinen Finger aufnimmt,
eine erste Ringfingertasche (826; 926) ist am dorsalen Abschnitt der Ringfingerhülse (886; 986) vorgesehen und so gestaltet, dass sie die erste Ausrichtungsführung für den gebogenen Ringfinger aufnimmt, und
eine erste Mittelfingertasche (825; 925) ist am dorsalen Abschnitt der Mittelfingerhülse (885; 985) vorgesehen und so gestaltet, dass sie die erste Ausrichtungsführung für den gebogenen Mittelfinger aufnimmt.

5. Trainingsvorrichtung nach Anspruch 2, wobei
die kleine Fingerhülse (887; 987) hat eine Öffnung, die so gestaltet ist, dass die Spitze des kleinen Fingers (87) freiliegt,
die Ringfingerhülse (886; 986) hat eine Öffnung, die so gestaltet ist, dass die Spitze des Ringfingers (86) freiliegt, und
die Mittelfingerhülse (885; 985) hat eine Öffnung, die so gestaltet ist, dass die Spitze des Mittelfingers (85) freiliegt.

6. Die Trainingsvorrichtung nach Anspruch 2 umfasst weiter
einen Daumenauschnitt (820; 989) und einen Zeigefingerauschnitt (886; 988).

7. Trainingsvorrichtung nach Anspruch 3 umfassend weiter
eine zweite Ausrichtungsführung für den gebogenen kleinen Finger und eine zweite Ausrichtungsführung für den gebogenen Ringfinger jeweils in Taschen an der palmaren Seite des Abschnitts des kleinen Fingers bzw. des Abschnitts des Ringfingers des Trainingshandschuhs eingeführt werden.

8. Trainingsvorrichtung nach Anspruch 2 umfassend weiter
eine Hülse für den kleinen Finger (887; 987), so dass die erste Ausrichtungsführung für den gebogenen kleinen Finger abnehmbar an der dorsalen Seite der Hülse für den kleinen Finger befestigt ist;
eine Ringfingerhülse (886; 986), so dass die erste Ausrichtungsführung für den gebogenen Ringfinger abnehmbar an der dorsalen Seite der Ringfingerhülse befestigt ist; und eine Mittelfingerhülse (885; 985), so dass die Ausrichtungsführung für den Mittelfinger abnehmbar an der dorsalen Seite der Mittelfingerhülse befestigt ist.

9. Trainingsvorrichtung nach Anspruch 2 umfassend weiter
eine Hülse für den kleinen Finger, so dass die erste Ausrichtungsführung für den gebogenen kleinen Finger abnehmbar an der palmaren Seite der Hülse für den kleinen Finger befestigt ist;
eine Ringfingerhülse, so dass die erste Ausrichtungsführung für den gebogenen Ringfinger abnehmbar an der palmaren Seite der Ringfingerhülse befestigt ist; und
eine Mittelfingerhülse, so dass die erste Mittelfingerausrichtungsführung abnehmbar an der palmaren Seite der Mittelfingerhülse befestigt ist.

10. Trainingsvorrichtung nach Anspruch 2 umfassend weiter
eine Hülse für den kleinen Finger, so dass
die erste Ausrichtungsführung für den gebogenen kleinen Finger abnehmbar an der dorsalen Seite der Hülse für den kleinen Finger befestigt ist;
eine zweite Ausrichtungsführung für den gebogenen kleinen Finger abnehmbar an der palmaren Seite der Hülse für den kleinen Finger befestigt ist;
eine Ringfingerhülse, so dass die erste Ausrichtungsführung für den gebogenen Ringfingers abnehmbar an der dorsalen Seite der Ringfingerhülse befestigt ist; und
eine zweite Ausrichtungsführung für den gebogenen kleinen Finger abnehmbar an der palmaren Seite der Ringfingerhülse befestigt ist;

11. Trainingsvorrichtung nach Anspruch 2 umfassend weiter
eine Hülse für den kleinen Finger (887; 987), so dass die erste Ausrichtungsführung für den gebogenen kleinen Finger an der Hülse für den kleinen Finger angebracht ist;
eine Ringfingerhülse (886; 986), so dass die erste Ausrichtungsführung für den gebogenen Ringfinger an der Ringfingerhülse angebracht ist;
eine Mittelfingerhülse (885; 985), so dass die erste Ausrichtungsführung für den Mittelfinger an der Mittelfingerhülse angebracht ist.

12. Trainingsvorrichtung nach Anspruch 1, wobei
die erste Ausrichtungsführung für den gebogenen kleinen Finger, die erste Ausrichtungsführung für den gebogenen Ringfinger und die erste Ausrichtungsführung für den gebogenen Mittelfinger sind als eine erste Form vorgesehen.

13. Trainingsvorrichtung nach Anspruch 12, wobei
die erste Form ist an der dorsalen Seite von mindestens einem der folgenden Finger befestigt: der kleine Finger, der Ringfinger oder der Mittelfinger.

14. Trainingsvorrichtung nach Anspruch 12, wobei
die erste Form ist an der palmaren Seite von mindestens einem der folgenden Finger befestigt: der kleine Finger, der Ringfinger oder der Mittelfinger.

15. Trainingsvorrichtung nach Anspruch 13 umfassend weiter
eine zweite Form umfassend
eine zweite Ausrichtungsführung für den gebogenen kleinen Finger, die die Biegung eines kleinen Fingers an einem ersten Knöchel, einem zweiten Knöchel und einem dritten Knöchel konturiert;
eine zweite Ausrichtungsführung für den gebogenen Ringfinger, die die Biegung eines Ringfingers an einem ersten Knöchel, einem zweiten Knöchel und einem dritten Knöchel konturiert, so dass die zweite Form an der palmare Seite von mindestens einem der folgenden Finger befestig ist: der kleine Finger oder der Ringfinger.

## Revendications

1. Dispositif d'alignement des doigts (800 ; 900) pour le positionnement de doigt d'un auriculaire (87) et de l'extrémité de l'auriculaire (872), de l'annulaire (86) et de l'extrémité de l'annulaire (862), et du majeur (85) et de l'extrémité du majeur (852), le dispositif comprenant:
une partie de l'auriculaire (887 ; 987) comprenant un premier guide d'alignement de l'auriculaire plié qui contourne le pliage d'un auriculaire au niveau d'une première articulation, d'une deuxième articulation et d'une troisième articulation;
une partie de l'annulaire (886 ; 986) comprenant un premier guide d'alignement de l'annulaire plié qui contourne le pliage d'un annulaire au niveau d'une première articulation, d'une deuxième articulation et d'une troisième articulation; et
une partie du majeur (885 ; 985) comprenant un premier guide d'alignement du majeur (945); **caractérisé en ce que** le dispositif est un dispositif d'entraînement à l'alignement des doigts (800 ; 900) pour enseigner le positionnement correct des doigts lors de l'exécution d'une procédure d'épilation au sucre cosmétique, et **en ce que** le guide d'alignement de l'auriculaire plié, le guide d'alignement de l'annulaire plié, et le guide d'alignement du majeur sont configurés pour positionner l'extrémité de l'auriculaire (872), l'extrémité de l'annulaire (862) et l'extrémité du majeur (852) le long d'une ligne lorsque l'extrémité de l'auriculaire, celle de l'annulaire et celle du majeur sont espacées l'une de l'autre.

2. Le dispositif d'entraînement à l'alignement des doigts de la revendication 1, dans lequel le dispositif d'entraînement est un gant d'entraînement comprenant
un manchon pour l'auriculaire (887 ; 987) ayant une partie dorsale et une partie palmaire,
un manchon pour l'annulaire (886 ; 986) ayant une partie dorsale et une partie palmaire, et
un manchon pour le majeur (885 ; 985) ayant une partie dorsale et une partie palmaire.

3. Le dispositif d'entraînement à l'alignement des doigts de la revendication 2 dans lequel
le premier guide d'alignement de l'auriculaire plié, le premier guide d'alignement de l'annulaire et le premier guide d'alignement du majeur (945) sont des inserts de doigt de gant séparés,
une première poche pour l'auriculaire (827 ; 927) est disposée sur la partie dorsale du manchon pour l'auriculaire (887 ; 987) et configurée pour accepter le premier guide d'alignement de l'auriculaire plié,
une premère poche pour l'annulaire (826 ; 926) est disposée sur la partie dorsale du manchon pour l'annulaire (886 ; 986) et configuré pour accepter le premier guide d'alignement de l'annulaire plié, et
une premère poche pour le majeur (825 ; 925) est disposée sur la partie dorsale du manchon pour le majeur (885 ; 985) et configurée pour accepter le premier guide d'alignement du majeur plié.

4. Le dispositif d'entraînement à l'alignement des doigts de la revendication 2 dans lequel
le premier guide d'alignement de l'auriculaire plié, le premier guide d'alignement de l'annulaire plié et le premier guide d'alignement du majeur (945) sont des inserts de doigt de gant séparés,
une première poche pour l'auriculaire (827 ; 927) est disposée sur la partie palmaire du manchon pour l'auriculaire (887 ; 987) et configurée pour accepter le premier guide d'alignement de l'auriculaire plié,
une première poche pour l'annulaire (826 ; 926) est disposée sur la partie dorsale du manchon pour l'annulaire (886 ; 986) et configurée pour accepter le premier guide d'alignement de l'annulaire plié, et
une première poche pour le majeur (825 ; 925) est disposée sur la partie dorsale du manchon pour le majeur (885 ; 985) et configurée pour accepter le premier guide d'alignement du majeur plié.

5. Le dispositif d'entraînement de la revendication 2 dans lequel
le manchon pour l'auriculaire (887 ; 987) a une ouverture configurée pour exposer l'extrémité d'un auriculaire (87),
le manchon pour l'annulaire (886 ; 986) a une ouverture configurée pour exposer l'extrémité d'un annulaire (86), et
le manchon pour le majeur (885 ; 985) a une ouverture configurée pour exposer l'extrémité d'un majeur (85).

6. Le dispositif d'entraînement de la revendication 2 comprend en outre
une découpe pour le pouce (820 ; 989) et une découpe pour l'index (886 ; 988).

7. Le dispositif d'entraînement de la revendication 3 comprenant en outre un deuxième guide d'alignement de l'auriculaire plié et un deuxième guide d'alignement de l'annulaire plié sont insérés chacun dans des poches disposées sur la face palmaire de la partie de l'auriculaire et de l'annulaire, respectivement, du gant d'entraînement.

8. Le dispositif d'entraînement de la revendication 2 comprenant en outre
un manchon pour l'auriculaire (887 ; 987) de sorte que le premier guide d'alignement de l'auriculaire plié est attaché de manière amovible à la face dorsale du manchon pour l'auriculaire;
un manchon pour l'annulaire (886 ; 986) de sorte que le premier guide d'alignement de l'annulaire plié est attaché de manière amovible à la face dorsale du manchon pour l'annulaire; et
un manchon pour le majeur (885 ; 985) de sorte que le premier guide d'alignement du majeur est attaché de manière amovible à la face dorsale du manchon pour le majeur.

9. Le dispositif d'entraînement de la revendication 2 comprenant en outre
un manchon pour l'auriculaire de sorte que le premier guide d'alignement de l'auriculaire plié est attaché de manière amovible à la face palmaire du manchon pour l'auriculaire;
un manchon pour l'annulaire de sorte que le premier guide d'alignement de l'annulaire plié est attaché de manière amovible à la face palmaire du manchon pour l'annulaire; et
un manchon pour le majeur de sorte que le guide d'alignement du premier premier guide d'alignement du majeur est attaché de manière amovible à la face palmaire du manchon pour le majeur.

10. Le dispositif d'entraînement de la revendication 2 comprenant en outre
un manchon pour l'auriculaire de sorte que
le premier guide d'alignement de l'auriculaire plié est attaché de manière amovible à la face dorsale du manchon pour l'auriculaire, et
un deuxième guide d'alignement de l'auriculaire plié est attaché de manière amovible à la face palmaire du manchon pour l'auriculaire; et
un manchon pour l'annulaire de sorte que le premier guide d'alignement de l'annulaire plié est attaché de manière amovible à la face dorsale du manchon pour l'annulaire, et
un deuxième guide d'alignement de l'auriculaire plié est attaché de manière amovible à la face palmaire du manchon pour l'auriculaire.

11. Le dispositif d'entraînement de la revendication 2 comprenant en outre
un manchon pour l'auriculaire (887 ; 987) de sorte que le premier guide d'alignement de l'auriculaire plié est affixé au manchon pour l'auriculaire;
un manchon pour l'annulaire (886 ; 986) de sorte que le premier guide d'alignement de l'annulaire plié est affixé au manchon pour l'annulaire; et
un manchon pour le majeur (885 ; 985) de sorte que le premier guide d'alignement du majeur est affixé au manchon pour le majeur.

12. Le dispositif d'entraînement de la revendication 1 dans lequel
le premier guide d'alignement de l'auriculaire plié, le premier guide d'alignement de l'annulaire plié et le premier guide d'alignement du majeur sont fournis en tant que première forme.

13. Le dispositif d'entraînement de la revendication 12 dans lequel
la première forme est attachée à la face dorsale d'au moins un parmi l'auriculaire, l'annulaire ou le majeur.

14. Le dispositif d'entraînement de la revendication 12 dans lequel
la première forme est attachée à la face palmaire d'au moins un parmi l'auriculaire, l'annulaire ou le majeur.

15. Le dispositif d'entraînement de la revendication 13 comprenant en outre
une deuxième forme comprenant
un deuxième guide d'alignement de l'auriculaire plié qui contourne le pliage d'un auriculaire au niveau d'une première articulation, d'une deuxième articulation et d'une troisième articulation, et
un deuxième guide d'alignement de l'annulaire plié qui contourne le pliage d'un annulaire au niveau d'une première articulation, d'une deuxième articulation et d'une troisième articulation,
de telle sorte que la deuxième forme soit attachée à la face palmaire d'au moins soit l'auriculaire soit l'annulaire.
